(19) **Europäisches Patentamt / European Patent Office / Office européen des brevets**

(11) **EP 3 618 806 B1**

(12) **EUROPEAN PATENT SPECIFICATION**

(45) Date of publication and mention
of the grant of the patent:
**01.03.2023  Bulletin 2023/09**

(21) Application number: **18728238.9**

(22) Date of filing: **04.05.2018**

(51) International Patent Classification (IPC):
**A61K 9/00** (2006.01)   **A61K 47/46** (2006.01)

(52) Cooperative Patent Classification (CPC):
**A61K 9/006; A61K 9/0053; A61K 47/46**

(86) International application number:
**PCT/IB2018/053128**

(87) International publication number:
**WO 2018/203303 (08.11.2018 Gazette 2018/45)**

(54) **COMPOSITON FOR THE TREATMENT OF APHTHAS AND MOUTH ULCERS**

ZUSAMMENSETZUNG ZUR BEHANDLUNG VON APHTHEN UND MUNDGESCHWÜREN

COMPOSITION POUR LE TRAITEMENT D'APHTES ET D'ULCÈRES BUCCAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **05.05.2017  IT 201700048750**

(43) Date of publication of application:
**11.03.2020  Bulletin 2020/11**

(73) Proprietor: **Farmaceutici Procemsa S.p.A.
10042 Nichelino (IT)**

(72) Inventors:
• **URAS, Giovanni
  10042 Nichelino (TO) (IT)**
• **SERTORIO, Alessandro
  10131 Torino (IT)**

(74) Representative: **Casciano, Lidia Giulia Rita et al
Studio Torta S.p.A.
Via Viotti, 9
10121 Torino (IT)**

(56) References cited:
EP-B1- 1 638 582        EP-B1- 2 646 036
WO-A2-2010/010346    CN-A- 105 496 833

• **Anonymous: "Rose damask - Rosa Damascena",
Palmarosa Essential Oil OEM Manufacturer from
Kanpur , 9 January 2018 (2018-01-09),
XP002777087, Retrieved from the Internet:
URL:https://www.indiamart.com/proddetail/r
ose-damask-rosa-damascena-11590022597.html
[retrieved on 2013-01-01]**
• **JH LEE ET AL: "The efficacy of topical
0.2% hyaluronic acid gel on recurrent oral ulcers",
JOURNAL OF THE EUROPEAN ACADEMY OF
DERMATOLOGY AND VENEREOLOGY : JEADV
NOV 2009,, vol. 22, no. 5, 1 May 2008 (2008-05-01),
pages 590-595, XP009502595, ISSN: 1468-3083,
DOI: 10.1111/J.1468-3083.2007.02564.X
[retrieved on 2007-12-17]**
• **Cheryll Williams: "Medicinal plants in Australia,
An Antipodean Apothecary", 1 January 2013
(2013-01-01), Rosenberg Publishing Pty Ltd,
XP002777089, ISBN: 1925078086 vol. 4, page 36,
first paragraph**

Remarks:
The file contains technical information submitted after
the application was filed and not included in this
specification

EP 3 618 806 B1

**Description**

*PRIORITY CLAIM*

*This application claims priority from* Italian Patent Application No. 102017000048750, filed on 05/05/2017.

TECHNICAL FIELD

The present invention relates to a composition for the treatment of aphthas and mouth ulcers and as adjuvant during children's teething.

BACKGROUND ART

The deciduous dentition, which starts to be replaced after around 6 years after birth with the appearance of permanent dentition that ends in adulthood (18-25 years old), is a milestone for the growth and development of infants.

There is no exact rule, either regarding the age at which the deciduous dentition starts or the type of dental eruption, of associated symptoms and discomfort.

Usually, the first deciduous teeth appear after six months of age (start of the seventh month), but this can vary greatly: in some children the first tooth can be already be visible at three months, while for others it may appear after 12 months of age. If the deciduous teeth of a child appear early or late, the same will occur with the permanent teeth.

Usually the first teeth to appear are the lower central incisors (6-7 months of age) followed by the upper central incisors (7-8 months of age), the upper lateral incisors (8-9 months of age) and the lower lateral incisors (10-11 months of age). The first molars normally appear between 14 and 18 months of age, while the first canines appear after 18 months of age. The deciduous dentition is completed at 24-30 months of age, with the appearance of the second molars (for a total of 20 teeth).

As already mentioned, there are many exceptions to the rule, and therefore in some children the upper teeth can appear before the lower ones, without this being a symptom or a sign of a particular physiological condition.

The severity and duration of the symptoms that children perceive during dentition varies greatly: for some the appearance of deciduous teeth can be a very painful experience, while others suffer no noteworthy discomfort.

The symptoms linked to dentition generally occur in advance of appearance of the tooth and can be:

- pain: this is undoubtedly the most important and most frequent symptom (especially during the appearance of the first incisors and molars), caused by inflammation of the gingival mucosa, irritated by the cutting tooth pushing through to reach the surface;
- irritability: the inflammation and pain that results (increasing with each passing day) can make children more irritable and nervous;
- decrease in appetite/difficulty in feeding: gingival pain can intensify during feeding, meaning that children may refuse food during dentition (with a temporary slowing of increase in weight). Naturally, this creates a vicious circle that increases the child's irritability and the parents' frustration: a child that is not correctly fed will become increasingly hungry and inconsolable;
- reduction in sleep: the situation described above can worsen the ability to fall sleep and remain asleep for long periods; in particular, the deterioration of the sleep-wake cycle can destabilise the routine of the child and of the parents;
- excessive salivation and irritation of facial skin, in particular the chin area, due to continuous contact with the saliva continuously produced;
- cough, mainly dry, also linked to excessive salivation;
- desire to bite: this is without a doubt the phase in which children start to put any object in their mouth and bite it. The pressure against the object, generally made of rubber, relieves pain.

Other symptoms, such as diarrhoea or increase in temperature, even if temporary, cannot be linked with certainty to dentition; this could be a contributing factor.

The treatment of these symptoms (completely physiological, but at times tedious for children and parents) is an argument of relevance, particularly well-studied in the scientific literature.

For example, in a recent study conducted on a group of over 1,000 children, some risk factors that could favour the onset of painful symptoms were identified: parental smoking, birth by Caesarean section, vitamin deficiency during pregnancy and ethnic origin.

These results, although not immediately relevant to solve the problem, have proved that this condition is more complex than initially expected.

In this context, possible therapeutic approaches to the problem are numerous and range from specific pharmaceutical remedies to others of purely mechanical nature (such as teething rings, which children can bite on, to relieve discomfort). Although this latter solution is currently very widely used and the market offers objects that can be cooled and maintained at low temperature for long periods of time to offer a refreshing sensation, it was recently verified that the prolonged use of these objects can cause damage to developed teeth, not only of an orthodontic, but also of a structural nature. Moreover, the hygiene conditions of these objects are difficult to control.

On the other hand, the use of medicinal products is not necessarily recommended. Already in 2011, the use of benzocaine was reported by the FDA as a potential cause of a rare disease with potentially fatal adverse effects (methemoglobinemia).

In this context, the use of a non-pharmaceutical product with lenitive properties can be a good substitute for classic teething rings and with a lower risk profile.

Another disorder of the oral cavity capable of causing considerable pain are aphthas, better known as recurrent oral aphthous ulcers.

These are ulcerative pathologies of the oral mucosa that affect more than 20% of the population. The aetiology of this disease has still to be defined. Aphthas are protruding round or oval ulcers, surrounded by a bright red halo, on the smooth tissue of the mucosa. Almost all types of aphthas, including those of very small size, are capable of causing pain.

Untreated lesions generally last for 7-10 days and heal without leaving a scar. Generally, aphtha treatments are intended to relieve the symptoms, even if many types of therapy have been considered.

For example, analgesics for topical use have been employed to relieve symptoms and anti-inflammatory agents to reduce pathological changes, while anti-bacterial agents are employed to control microbial contamination and secondary infections. Anti-bacterial agents include antibiotics (tetracycline) and antiseptics (chlorhexidine).

Mouthwashes containing broad spectrum antibiotics are able to reduce the formation of new ulcers, following a treatment of 10 days. This effect is due to a decrease in oral microflora, which in turn reduces the effects of a secondary infection.

However, antibiotics have a potentially undesirable mycotic effect and can give rise to allergic reactions.

Anti-bacterial mouthwashes can provide some benefit in terms of pain control, reducing both the effects caused by a secondary infection and the duration of the ulcer. Chlorhexidine can reduce the total number of days of ulceration, but is not entirely effective on the incidence or severity thereof. Moreover, it frequently causes colour changes of the teeth and tongue and alters the sensation of taste.

Moreover, various products based on hyaluronic acid are known for treating aphthas, for example as described in EP1638582. However, none of these has proved satisfactory. EP2646036 discloses buccal compositions for preserving the oral mucosae (like mouth ulcers and inflamed gums) comprising choline alfoscerate, sodium hyaluronan and carrier. WO2010010346 discloses the use of a composition comprising hyaluronic acid in the preparation of an oral medicament for reducing algesia and inflammation associated with tooth eruption (teething) in combination with alternative plant extracts. The article of Cheryll Williams, Medicinal plants in Australia, An Antipodean Apothecary, Rosenberg Publishing Pty Ltd, (20130101),vol.4,p.36, describes a mouthwash containing a generic Rosedamascena used for treating aphthous stomatitis (cancer sore).

DISCLOSURE OF INVENTION

Therefore, the object of the present invention is to provide a new composition for the treatment of aphthas and to relieve the symptoms of dentition in children.

This object is achieved by the present invention, which relates to a composition for oral use according to claim 1 and to its uses according to claim 7 and 8.

BEST MODE FOR CARRYING OUT THE INVENTION

According to a first aspect of the invention there is provided a oral composition comprising hyaluronic acid with an average molecular weight ranging between 800,000 and 4,000,000 and a hydroglycerin extract of Rosa Damascena petals having a polyphenol content of at least 8g/l expressed as equivalents of caffeic acid.

Rosa Damascena is a hybrid derived from Rosa gallica and from Rosa moschata. It is a plant that is no longer found in the wild state. Its geographical origin is perhaps central Asia and it is grown for ornamental purposes in many parts of the world, due to its adaptability to different climates. Moreover, this plant has industrial applications that justify its large-scale growth (in particular in Turkey and Bulgaria). Its main uses are cosmetic use of the flower extract (it is used to produce "rose water") and use in the food industry as flavouring. The petals are edible and are a common ingredient in the traditional cuisine of Eastern European countries.

Advantageously, the addition of this component to the composition of the invention provide astringent properties.

In particular, the tannins contained therein are able to precipitate some proteins of the saliva giving the typical sensation of astringency in the mouth. This results in a contraction of the mucous membranes, which thus reduces the sensation of pain. Moreover, tannins make the outer layer of the mucosa impermeable, protecting the deeper layers of the buccal skin. By reducing the loss of liquid and preventing external damage, tannins facilitate tissue regeneration in the case of wounds or eruption of teeth.

Moreover, advantageously, the use of hydroglycerin extract of Rosa Damascena gives the composition a coloured appearance that allows correct application of the product, as it is more easily seen. Moreover, it improves its palatability and also has an antioxidant action. Finally, the polyphenols of rose extract have an antioxidant action also on the composition and antiradical action.

Hydroglycerin extract of Rosa Damascena is produced by extraction with a mixture of water and glycerol, preferably in a ratio of 50/50, of Rosa Damascena petals that are left to macerate at room temperature and subsequently pressed and filtered. Rose extract is preferably obtained according to the following parameters:

- Drug/Extract (D/E) ratio: from 1:5 to 1:3;
- Extraction solvent: glycerin/water mixture in a ratio from 40/60 to 60/40;

- Extraction temperature: from 20°C to 45°C;
- Extraction time: from 6 hours to 30 days;
- Extraction method: maceration and percolation, if necessary with the aid of ultrasound.

Preservatives, preferably ascorbic acid at 0.15% and potassium sorbate at 0.30%, can also be added.

Advantageously, the extract thus obtained has a polyphenol content of at least 8 g/l expressed as equivalents of caffeic acid.

According to an embodiment of the invention, the composition comprises from 0.01% to 0.4% by weight of hyaluronic acid, and from 0.5% to 10% by weight of hydroglycerin extract of Rosa Damascena, based on the total weight of the composition, preferably from 0.02% to 0.3% by weight of hyaluronic acid, and from 2% to 5% by weight of hydroglycerin extract of Rosa Damascena, based on the total weight of the composition.

The composition can also comprise one or more flavouring agents selected from the group consisting of xylitol, Chamomilla recutita, glycerol, sorbitol, mallow and aloe, preferably in an amount ranging between 0.5 and 70%.

The composition can also comprise one or more mucoadhesive and/or film-forming agents selected from the group consisting of pectin, aloe vera juice, Malva sylvestris extract, xanthan gum, polycarbophyl, alginates, cellulose derivatives, PVP, VA/VP copolymers, vegetable mucilages, and mixtures thereof, preferably in an amount ranging between 0.1 and 25% by weight, based on the total weight of the composition.

The composition can also comprise one or more thickening agents selected from the group consisting of xanthan gum, cellulose derivatives, alginates, gum arabic, other natural polymers, preferably in an amount ranging between 0.1 and 25% by weight, based on the total weight of the composition.

According to a further aspect of the invention, the oral composition described above can be used in the treatment of aphthas and mouth ulcers and as adjuvant during children's teething. Further features of the present invention will be more apparent from the description provided below of some purely illustrative and non-limiting examples.

Example 1

The following composition was prepared:

| APHTHAS GEL WITH ROSE EXTRACT | |
|---|---|
| Component | amount |
| Xylitol | 40 g |
| Hydroglycerin extract of Rosa Damascena buds | 4 g |
| High molecular weight sodium hyaluronate | 0.10 g |
| Xanthan gum | 3 g |
| Sodium benzoate | 0.1 g |
| Potassium sorbate | 0.1 g |
| Citric acid | qs to 100 g |

(continued)

| APHTHAS GEL WITH ROSE EXTRACT | |
|---|---|
| Component | amount |
| Demineralised water | qs to 100 g |

In a suitable container the water required in the formula is weighed and heated to 60 degrees Celsius. Upon reaching this temperature the xanthan gum is added and it is homogenised with a suitable turbine instrument, until a uniform gel is obtained. The xylitol is added in portions and it is taken to room temperature; separately the sodium hyaluronate is dispersed in the hydroglycerin extract of Rosa Damascena buds, using suitable equipment (such as ultra turrax) until uniform gelling and the established amount of preservatives is added thereto.

The semi-finished product thus obtained is added to the water, gum and xylitol gel and it is uniformed to obtain a bright glossy gelling product with uniform colour, characteristic rose flavour and aroma. Finally, it is acidified to a pH below 5.

Example 2

Mucoadhesion Test

The object of this test consists in the quantitative assessment of the mucoadhesive property of the product of example 1 through analysis of the detachment kinetics of the sample from an inclined steel surface, without and with a mucin biofilm. Effective mucoadhesion is determined by the delay in sliding of the sample and in detachment from the surface caused by the presence of mucin.

The steel surface is positioned horizontally and the temperature is set 60' before the start of the run. 2 ml of sample (or of deionised water for the controls) is placed at the predetermined distance on the surface and left to rest for 2'. To perform the test, the surface is inclined to 45° and the detached sample is weighed at the end of run. The sample falls into a Petri dish and is weighed with automatic recordings every 60". An Ohaus Explorer Pro analytic balance connected to a Bixolon thermal printer is used for the gravimetric readings.

Each run is carried out in triplicate according to the following method:

- Deionised water without mucin
- Deionised water with mucin
- Sample without mucin
- Sample with mucin

The surface is carefully cleaned and prepared prior to each run. For runs with mucin, the surface is pre-treated by applying 3 ml of 8% suspension in water of porcine gastric mucin type II (Sigma Aldrich) until a uniform film is formed.

The sample run conditions are as follows:

- Temperature: 36°C.
- Surface inclination: 45°.
- Length of the run surface: 10 cm.
- Duration of the run: 60".

In order to calculate mucoadhesion, the averages of the weighings recorded for each unit of time considered, with their standard deviations, were considered. Each average is compared to the total weight of the sample applied to obtain an adhesion curve in time.

The adhesion percentage values (percentage of sample adhering to the mucin), calculated for each time, were obtained using the following formula:

$$\% \text{ Adhesion } \% = (P0-Pt)/P0 * 100$$

where

P0 = weight of the sample applied at T=0

Pt = weight of the detached sample at the time t

To be considered mucoadhesive, a sample must show an adhesion compared to the control without mucin significantly greater (p<0.05) to that shown by the water, which interacts with the protein by hygroscopic absorption only.

The results are set down in Table 1 in terms of percentage of adhesion in time, plus or minus the standard deviation.

Table 1

| Time (min) | 1 | 2 | 3 | 4 | 5 | 10 | 15 | 20 |
|---|---|---|---|---|---|---|---|---|
| Water (%) | 13.17 | 12.33 | 12.33 | 12.33 | 12.33 | 12.33 | 12.33 | 12.33 |
| (deviation ±%) | 2.18 | 1.33 | 1.30 | 1.52 | 1.33 | 1.52 | 1.56 | 1.81 |
| Water+mucin (%) | 23.00 | 22.50 | 22.83 | 23.00 | 23.17 | 23.50 | 24.17 | 24.83 |
| (deviation ±%) | 1.50 | 1.15 | 1.15 | 1.15 | 1.15 | 0.75 | 0.50 | 0.25 |
| Sample (%) | 72.67 | 56.33 | 51.33 | 51.50 | 51.50 | 45.00 | 41.67 | 39.00 |
| (deviation ±%) | 24.55 | 7.97 | 0.58 | 0.58 | 0.50 | 6.06 | 0.58 | 5.63 |
| Sample +mucin (%) | 100 | 100 | 81.67 | 72.17 | 71.83 | 61.83 | 58.67 | 59.17 |
| (deviation ±%) | 0 | 0 | 15.09 | 2.29 | 2.52 | 9.07 | 4.04 | 4.04 |
| | | | | | | | | |
| Time (min) | 25 | 30 | 35 | 40 | 45 | 50 | 55 | 60 |
| Water (%) | 12.33 | 12.33 | 12.33 | 12.33 | 12.33 | 12.33 | 12.33 | 12.33 |
| (deviation ±%) | 1.64 | 1.56 | 1.30 | 1.09 | 1.75 | 1.50 | 2.00 | 2.30 |
| Water+mucin (%) | 24.83 | 25.00 | 24.50 | 24.50 | 24.00 | 23.67 | 24.33 | 24.83 |
| (deviation ±%) | 0.50 | 0.90 | 1.25 | 1.50 | 1.50 | 2.14 | 2.18 | 2.60 |
| Sample (%) | 39.17 | 39.17 | 39.33 | 36.50 | 33.67 | 33.83 | 34.00 | 34.17 |
| (deviation ±%) | 5.77 | 5.77 | 5.92 | 5.68 | 0.58 | 0.76 | 0.87 | 0.58 |
| Sample +mucin (%) | 59.00 | 59.50 | 59.17 | 59.33 | 59.50 | 59.67 | 60.00 | 60.00 |
| (deviation ±%) | 3.91 | 3.91 | 3.62 | 3.75 | 3.46 | 3.62 | 3.91 | 3.91 |

We can see how between 3' and 60' the sample tested has a percentage of mucoadhesion ranging between 60 and 810, against a percentage of deionised water of 12.33%. The average percentage of adhesion of the sample to the sheet without mucin is 40%.

Example 3

To verify the antibacterial activity of the composition of the invention, the composition of example 1 (80 g) was inoculated in different bacterial cultures. The results are illustrated in Table 2.

Table 2

| | ST. AUREUS ATCC 6538 | P. AERUGINOSA ATCC 9027 | C. ALBICANS ATCC 10231 | A. BRASILIENSIS ATCC 16404 |
|---|---|---|---|---|
| INOCULATION CFU/G | 3.00E+05 | 5.00E+05 | 4.00E+05 | 3.00E+05 |
| INOCULATION LOG | 5.48 | 5.70 | 5.60 | 5.48 |
| RECOVERY AFTER 2 DAYS | <10 | <10 | / | / |
| REDUCTION AFTER 2 DAYS (LOG) | >4 | >4 | / | / |

(continued)

|  | ST. AUREUS ATCC 6538 | P. AERUGINOSA ATCC 9027 | C. ALBICANS ATCC 10231 | A. BRASILIENSIS ATCC 16404 |
|---|---|---|---|---|
| RECOVERY AFTER 7 DAYS | <10 | <10 | / | / |
| REDUCTION AFTER 7 DAYS (LOG) | >4 | >4 | / | / |
| RECOVERY AFTER 14 DAYS | / | / | <10 | <10 |
| REDUCTION AFTER 14 DAYS (LOG) | / | / | >4 | >4 |
| RECOVERY AFTER 28 DAYS | <10 | <10 | <10 | <10 |
| REDUCTION AFTER 28 DAYS (LOG) | >4 | >4 | >4 | >4 |

The results are in conformity compared with the required acceptance criteria (BACTERIA: 2 DAY 2 LOG RED., 7 DAYS 3 LOG RED., 28 DAYS NO GROWTH - FUNGI: 14 DAYS 2 LOG RED., 28 DAYS NO GROWTH).

**Claims**

1. A composition for oral use comprising hyaluronic acid with an average molecular weight comprised between 800,000 and 4,000,000, and hydroglycerin extract of Rosa Damascena petals having a polyphenol content of at least 8 g/l expressed as equivalents of caffeic acid.

2. A composition according to claim 1, **characterised in that** it comprises from 0.01% to 0.4% by weight of hyaluronic acid, and from 0.5% to 10% by weight of hydroglycerin extract of Rosa Damascena, based on the total weight of the composition.

3. A composition according to any one of claims 1 or 2, **characterised in that** it comprises from 0.02% to 0.3% by weight of hyaluronic acid, and from 2% to 5% by weight of hydroglycerin extract of Rosa Damascena, based on the total weight of the composition.

4. A composition according to any one of claims 1 to 3, **characterised in that** it comprises one or more flavouring agents selected from the group consisting of xylitol, Chamomilla recutita, glycerol, sorbitol, mallow, aloe, and mixtures thereof, preferably in an amount comprised between 0.5 and 70%.

5. A composition according to any one of claims 1 to 4 **characterised in that** it comprises one or more mucoadhesive and/or film-forming agents selected from the group consisting of pectin, aloe vera juice, Malva sylvestris extract, xanthan gum, polycarbophyl, alginates, cellulose derivatives, PVP, VA/VP copolymers, vegetable mucilages, and mixtures thereof, preferably in an amount comprised between 0.1 and 25% by weight, based on the total weight of the composition.

6. A composition according to any one of claims 1 to 5 **characterised in that** it comprises one or more thickening agents selected from the group consisting of xanthan gum, cellulose derivatives, alginates, gum arable, other natural polymers, preferably in an amount comprised between 0.1 and 25% by weight, based on the total weight of the composition.

7. A composition according to any one of claims 1 to 6 for use in the treatment of aphthae and mouth ulcers.

8. A composition according to any one of claims 1 to 6 for use as adjuvant during children's teething.

**Patentansprüche**

1. Eine Zusammensetzung zur oralen Verwendung umfassend Hyaluronsäure mit einem durchschnittlichen Molekulargewicht zwischen 800.000 und 4.000.000 und Hydroglycerinextrakt aus Rosa Damascena-Blütenblättern mit einem Polyphenolgehalt von mindestens 8 g/l, ausgedrückt als Äquivalente von Kaffeesäure.

2. Eine Zusammensetzung nach Anspruch 1, **dadurch gekennzeichnet, dass** sie 0,01 bis 0,4 Gew.-% Hyaluronsäure und 0,5 bis 10 Gew.-% Hydroglycerinextrakt von Rosa Damascena, bezogen auf das Gesamtgewicht der Zusammensetzung, umfasst.

3. Eine Zusammensetzung nach einem der Ansprüche 1 oder 2, **dadurch gekennzeichnet, dass** sie 0,02 bis 0,3 Gew.-% Hyaluronsäure und 2 bis 5 Gew.-% Hydroglycerinextrakt von Rosa Damascena, bezogen auf das Gesamtgewicht der Zusammensetzung, umfasst.

4. Eine Zusammensetzung nach einem der Ansprüche 1 bis 3, **dadurch gekennzeichnet, dass** sie einen oder mehrere Aromastoffe umfasst, die ausgewählt sind aus der Gruppe bestehend aus Xylit, Chamomilla recutita, Glycerin, Sorbit, Malve, Aloe und Gemischen davon, vorzugsweise in einer Menge zwischen 0,5 und 70%.

5. Eine Zusammensetzung nach einem der Ansprüche 1 bis 4, **dadurch gekennzeichnet, dass** sie ein oder mehrere mukoadhäsive und/oder filmbildende Mittel umfasst, die ausgewählt sind aus der Gruppe bestehend aus Pektin, Aloe Vera-Saft, Malva sylvestris-Extrakt, Xanthangummi, Polycarbophyl, Alginaten, Cellulosederivaten, PVP, VA/VP-Copolymeren, pflanzlichen Schleimstoffen und Gemischen davon, vorzugsweise in einer Menge zwischen 0,1 und 25 Gew.-%, bezogen auf das Gesamtgewicht der Zusammensetzung.

6. Eine Zusammensetzung nach einem der Ansprüche 1 bis 5, **dadurch gekennzeichnet, dass** sie ein oder mehrere Verdickungsmittel umfasst, die ausgewählt sind aus der Gruppe bestehend aus Xanthangummi, Cellulosederivaten, Alginaten, Gummi arabicum, anderen natürlichen Polymeren, vorzugsweise in einer Menge zwischen 0,1 und 25 Gew.-%, bezogen auf das Gesamtgewicht der Zusammensetzung.

7. Eine Zusammensetzung nach einem der Ansprüche 1 bis 6 zur Verwendung in der Behandlung von Aphthen und Mundgeschwüren.

8. Eine Zusammensetzung nach einem der Ansprüche 1 bis 6 zur Verwendung als Hilfsmittel beim Zahnen von Kindern.

**Revendications**

1. Composition pour usage oral comprenant de l'acide hyaluronique ayant un poids moléculaire moyen compris entre 800 000 et 4 000 000, et un extrait hydroglycériné de pétales de Rosa Damascena ayant une teneur en polyphénols d'au moins 8 g/l exprimée en équivalents d'acide caféique.

2. Composition selon la revendication 1, **caractérisée en ce qu'**elle comprend de 0,01% à 0,4% en poids d'acide hyaluronique, et de 0,5% à 10% en poids d'extrait hydroglycériné de Rosa Damascena, par rapport au poids total de la composition.

3. Composition selon l'une quelconque des revendications 1 ou 2, **caractérisée en ce qu'**elle comprend de 0,02% à 0,3% en poids d'acide hyaluronique, et de 2% à 5% en poids d'extrait hydroglycériné de Rosa Damascena, par rapport au poids total de la composition.

4. Composition selon l'une quelconque des revendications 1 à 3, **caractérisée en ce qu'**elle comprend un ou plusieurs agents aromatisants choisis dans le groupe comprenant le xylitol, la Chamomilla recutita, le glycérol, le sorbitol, la mauve, l'aloès, et des mélanges de ceux-ci, de préférence en une quantité comprise entre 0,5 et 70%.

5. Composition selon l'une quelconque des revendications 1 à 4, **caractérisée en ce qu'**elle comprend un ou plusieurs agents mucoadhésifs et/ou filmogènes choisis dans le groupe comprenant la pectine, le jus d'Aloe Vera, l'extrait de Malva sylvestris, la gomme xanthane, le polycarbophile, les alginates, les dérivés de cellulose, le PVP, les copolymères VA/VP, les mucilages végétaux, et des mélanges de ceux-ci, de préférence en une quantité comprise entre 0,1 et 25% en poids, par rapport au poids total de la composition.

6. Composition selon l'une quelconque des revendications 1 à 5, **caractérisée en ce qu'**elle comprend un ou plusieurs agents épaississants choisis dans le groupe comprenant la gomme xanthane, les dérivés de cellulose, les alginates, la gomme arabique, d'autres polymères naturels, de préférence en une quantité comprise entre 0,1 et 25% en poids, par rapport au poids total de la composition.

7. Composition selon l'une quelconque des revendications 1 à 6 pour une utilisation dans le traitement d'aphtes et d'ulcères buccaux.

8. Composition selon l'une quelconque des revendications 1 à 6 pour une utilisation comme adjuvant lors de poussée des dents d'enfants.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 102017000048750
- EP 1638582 A

- EP 2646036 A
- WO 2010010346 A

**Non-patent literature cited in the description**

- **CHERYLL WILLIAMS.** Medicinal plants in Australia, An Antipodean Apothecary. Rosenberg Publishing Pty Ltd, vol. 4, 36